# EUROPEAN PATENT SPECIFICATION

(11) **EP 2 967 453 B1**
(45) Date of publication and mention of the grant of the patent: **13.05.2020**
(21) Application number: 14773376.0
(22) Date of filing: 10.03.2014
(51) Int. Cl.: A61B 5/145, G01N 33/49, G06Q 10/00

(54) **CONTINUOUS GLUCOSE MONITORING ON-BODY SENSOR HAVING A VISUAL DISPLAY**
KONTINUIERLICHE GLUCOSEÜBERWACHUNG AUF EINEM KÖRPERSENSOR MIT EINER VISUELLEN ANZEIGE
CAPTEUR CORPOREL DE SURVEILLANCE CONTINUE DU GLUCOSE

(30) Priority: 14.03.2013 US 201361782019 P
(43) Date of publication of application: 20.01.2016
(60) Divisional application: 20158136.0
(73) Proprietor: Becton, Dickinson and Company, Franklin Lakes, NJ 07417-1880 (US)
(72) Inventor: DI RESTA, Ellen, Arlington, MA 02476 (US); PRUDDEN, John, Manchester, MA 01944 (US); SALEMME, James, Billerica, MA 01821 (US); GUNDLACH, Jack, Acton, MA 01720 (US); SULLIVAN TREACY, Ann, South Yarmouth, MA 02664 (US); LINNANE, Jennifer, Melrose, MA 02176 (US)
(74) Representative: dompatent von Kreisler Selting Werner - Partnerschaft von Patent- und Rechtsanwälten mbB
(86) International application number: PCT/US2014/022613
(87) International publication number: WO 2014/159229

(56) References cited:
- WO-A2-2010/104952
- US-A1- 2006 142 651
- US-A1- 2010 063 438
- US-A1- 2011 184 264
- US-A1- 2011 213 225
- US-A1- 2011 230 735
- US-A1- 2011 288 379
- US-A1- 2012 157 801
- US-A1- 2012 245 447
- US-A1- 2012 302 844
- US-A1- 2012 323 094

## Description

### Cross-Reference to Related Application

This application claims the benefit under 35 U.S.C. § 119 (e) of U.S. Provisional Application No. 61/782,019, filed on March 14, 2013, in the U.S. Patent and Trademark Office.

### Field of the Invention

The present invention relates generally to continuous glucose monitoring (CGM) devices used to continuously monitor subcutaneous glucose using optical interrogation of a glucose binding protein (GBP) to determine the concentration of glucose in a user. More particularly, the present invention relates to on-body sensors (OBS) incorporating CGM devices and having covers with integrated output displays.

### Background of the Invention

In patients with diabetes, glucose levels need to be monitored to maintain a healthy balance of glucose in the body. Glucose levels can be monitored by GBP coated sensors such as on-body CGM devices. CGM devices can have a needle or probe that is inserted into the tissue of a user to measure the glucose levels in the surrounding tissue fluid.

Conventionally, on-body CGM devices are usually small and configured to be secured to the skin of a user's abdomen during each sensor wear period. A transmitter is incorporated into the CGM device and communicates with a handheld receiver. The data collected by the CGM device is transferred to the receiver at intervals throughout the wear period.

Without a display incorporated into the OBS, a user must carry a separate device to inspect the information obtained by and/or processed by the CGM device. Therefore, a patient often does not have the benefit of knowing current glucose levels or trends due to not having a data receiving device to receive, process and display the data from the CGM device.

It is also important to maintain a low profile CGM device in order to reduce interference with the activities of the user and reduce possible skin irritation. Without a low profile CGM device normal body movement of a user can cause unwanted micro-motion of the needle or probe which can compromise the data collected by the CGM device. Additionally, the shape and exterior configuration of the on-body CGM device can catch on a user's clothing causing additional irritation to the user and even malfunction of the device itself.

### Summary of the Invention

An object of illustrative embodiments of the present invention is to substantially address the above and other concerns, and provide improved structure to OBS devices.

Another object of illustrative embodiments of the present invention is to provide an OBS device that will provide an on-body output display.

Another object of illustrative embodiments of the present invention is to provide an on-body display that can be conveniently inspected by a user in multiple positions.

Another object of illustrative embodiments of the present invention is to provide an on-body display that maintains an overall low profile of the OBS such that interference with the movements of a user is minimized.

Another object of illustrative embodiments of the present invention is to allow a patient to move freely while maintaining the proper positioning of the OBS device.

Another object of illustrative embodiments of the present invention is to enable the OBS device to flex and move with the user, but reduce micro-motions of the needle that can cause malfunction of the OBS and injure the user.

These and other objects are substantially achieved by providing an on-body device as defined in claim 1 wherein the cover includes an integrated on-body output display having a reduced profile while maintaining structural and positional integrity, thereby improving the effectiveness, comfort, durability and securement of the OBS device. A device according to the preamble of claim 1 is known from document US2006/142651.

### Brief Description of the Drawings

The various objects, advantages and novel features of illustrative embodiments of the present invention will be more readily appreciated from the following detailed description when read in conjunction with the appended drawings, in which:
Fig. 1 is a cross-sectional view of a CGM device in accordance with an illustrative embodiment of the present invention;
Fig. 2 is a schematic diagram of the CGM device of Fig. 1 including ray traces through an optical coupler, from a light-emitting diode (LED) to a fiber face;
Fig. 3 is a schematic diagram of the CGM device of Fig. 1 including ray traces through the optical coupler, from the fiber face to a photodiode;
Fig. 4 is an illustrative embodiment of an on-body cover and display for a CGM device;
Fig. 5 is another illustrative embodiment of an on-body cover and display for a CGM device;
Figs. 6 and 7 illustrate a user visually inspecting an illustrative on-body display of a CGM device; and
Fig. 8 is an illustrative embodiment of an on-body cover and display for a CGM device.

### Detailed Description of Illustrative Embodiments

As will be appreciated by one skilled in the art, there are numerous ways of carrying out the examples, improvements and arrangements of CGM devices disclosed herein. Although reference will be made to the illustrative embodiments depicted in the drawings and the following descriptions, the embodiments disclosed herein are not meant to be exhaustive of the various alternative designs and embodiments that are encompassed by the disclosed invention.

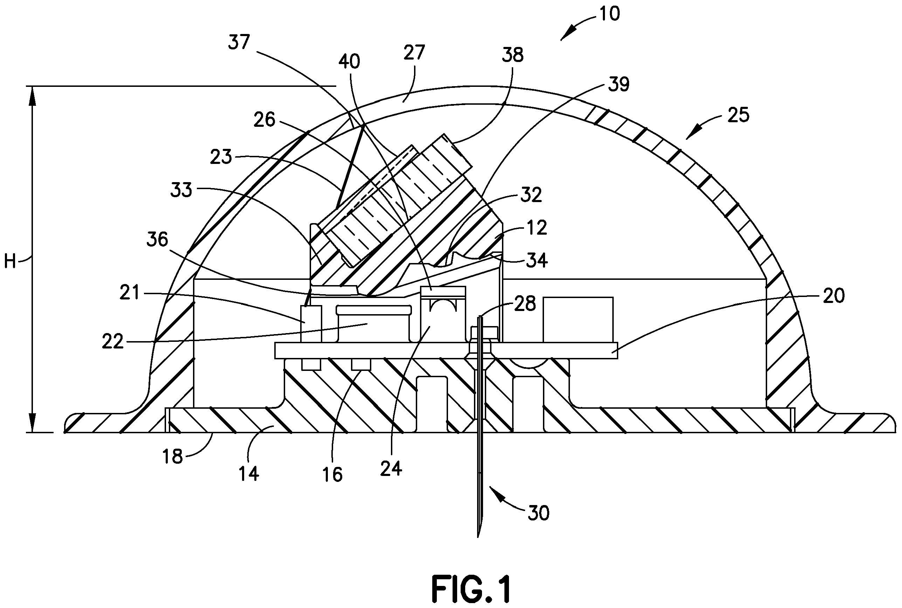

Figs. 1-3 illustrate an illustrative embodiment of an on-body CGM sensor 10 utilizing an optical coupler 12 in accordance with the present invention. The CGM sensor 10 includes a base 14 with a top surface 16 that supports the various components of the CGM sensor 10. A bottom surface 18 of the base 14 is used to support and adhere the CGM sensor to the skin of a user. For example, the bottom surface 18 of the base 14 can include an adhesive to adhere the CGM sensor to the skin of a user. A printed circuit board 20 is fixed to the top surface 16 of the base 14 and enables communication between a microcontroller 21, a photodiode 22 and LED 24, and an output display 27. A cover 25 substantially encloses the components of the CGM sensor 10 and is fixed to the base 14.

The LED 24 emits light that is selectively filtered by a filter 26 fixed to a top surface of the LED 24. The optical coupler 12 is positioned above the LED 24 and photodiode 22 and directs the light emitted from the LED 24 into a fiber 28 positioned adjacent to the LED 24. The fiber 28 runs through the length of a needle 30. The needle 30 is used to insert the fiber 28 into a user to provide contact between the fiber 28 and biomaterial, such as GBP, beneath the skin of the user. The GBP coats or is deposited on the end of the needle 30 and contacts blood or interstitial fluid (ISF) after insertion into the user.

The optical coupler 12 includes a plastic connector 33 having three integral lenses, an LED lens 32, a fiber lens 34 and a detector lens 36. The plastic connector also includes a pair of inclined glass mounting surfaces 37 and a mirrored surface 39 that reflects light emitted from the LED 24 through the fiber lens 34 and into the fiber 28 to transmit light to the GBP. The glass mounting surfaces 37 are configured to support and fix filters at a predetermined angle with respect to the photodiode 22, the LED 24 and the fiber 28. The plastic connector 31 can be manufactured as a single injection molded component, reducing the number of individual parts of the optical coupler 12 that need to be manufactured and assembled. The plastic connector 31 can also be formed by other desired manufacturing processes capable of forming a single unitary component.

The optical coupler 12 includes a first glass filter 38 and a second glass filter 40. The first glass filter 38 is fixed to the second glass filter 40 via gluing or another desired securing mechanism. The glued first and second glass filters 38 and 40 are also fixed or glued to the inclined glass mounting surfaces 37. After the first and second glass filters 38 and 40 are fixed together, only two components need to be positioned during assembly, the fixed glass filters 38 and 40 and the inclined surfaces of the 37 of the optical coupler 12. This simplified assembly reduces possible misalignment of components and potential failure of the CGM sensor 10. Additionally, by fixing the first and second glass filters 38 and 40 together and then directly fixing them to the inclined surfaces of the optical coupler 12, less light is lost and/or diffused during operation, thereby improving the efficiency of the optical coupler 12, as opposed to other known optical couplers that require the light to travel in and out of more open air spaces which cause increased inefficiency in light transfer.

The first glass filter 38 includes a first dichroic filter coating 42 on the surface of the glass filter 38 mounted to the glass mounting surfaces 37. The first dichroic filter coating 42 reflects the light wavelengths emitted by the LED and transmits emission light wavelengths emitted from the GBP via the fiber 28.

The second glass filter 40 includes a second dichroic filter coating 44 on the same surface that is mounted to the first glass filter 38. The second dichroic filter coating 44 reflects shorter emission wavelengths representing a signal band and transmits longer wavelengths representing a reference band. A mirror surface 46 is formed on the surface of the second glass filter 40 opposite to the surface mounted to the first glass filter 38. The mirrored surface 46 reflects all wavelengths, but is particularly used to reflect the long wavelengths transmitted by the second dichroic filter coating 44.

Microcontroller 21 is provided at least for operating and controlling the photodiode 22, LED 24 and output display 27. Microcontroller 21 is preferably fully programmable prior to installation within the CGM device to precisely control the operation of the photodiode 22 and data transmitted to the output display 27 via hardwired connection 23. The microcontroller 21 can also be programmable to manipulate and modify the type of data displayed on the output display 27. For example, the microcontroller 21 can transmit data to the output display 27 relating to a user's current glucose levels, glucose trends, CGM device malfunction notifications, when the output display 27 is illuminated or shut down, and glucose measurement intervals. Additional data processing and transmission can also be provided by the microcontroller 21.

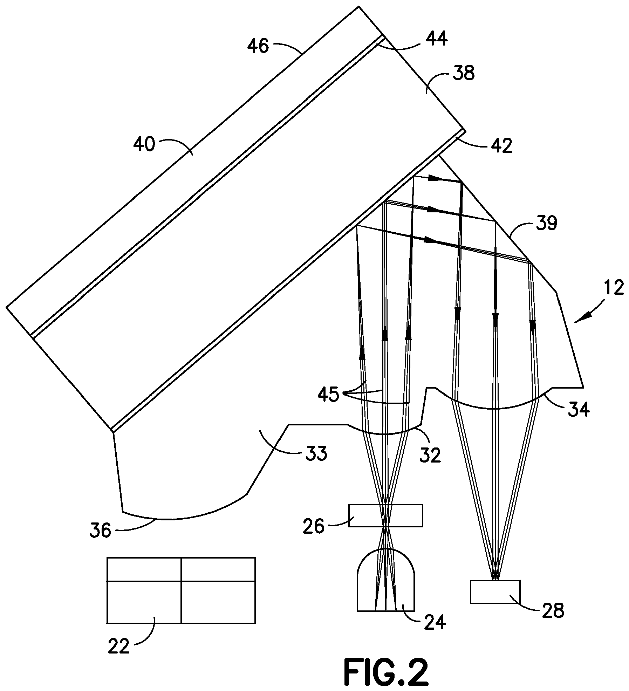

Fig. 2 illustrates a schematic diagram of the CGM sensor 10 in accordance with an illustrative embodiment of the present invention, including ray traces representing the light path from the LED 24 through the optical coupler 12 to the fiber 28 for illuminating the GBP in contact with an end of the fiber 28. Light 45 is first emitted from the LED 24 and filtered by the filter 26. The light 45 then travels through the LED lens 32 which focuses and directs the light 45 toward the first dichroic coating 42 which reflects the light 45 toward the mirrored surface 39 of the optical coupler 12. The mirrored surface 39 then reflects the light 45 toward the fiber lens 34 which focuses and transmits the light 45 toward the fiber 28 which illuminates the GBP (not shown).

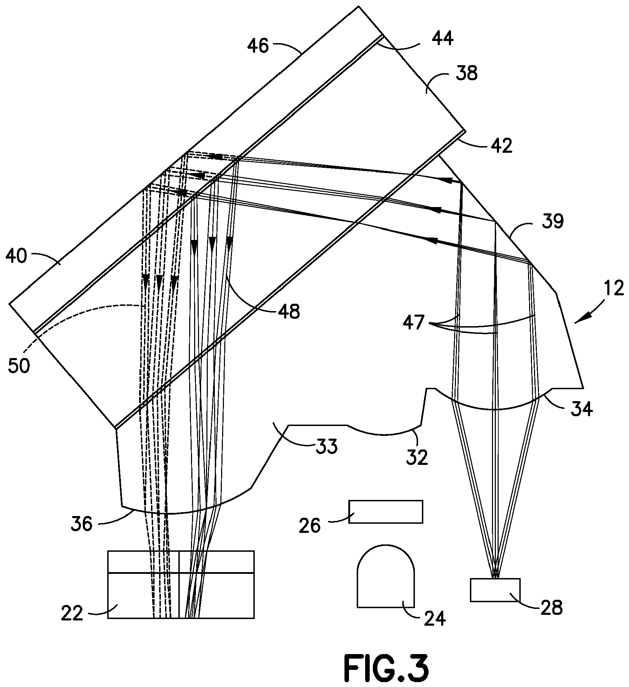

Fig. 3 illustrates a schematic diagram of the CGM sensor 10 in accordance with an illustrative embodiment of the present invention, including ray traces representing the light path from the fiber 28 through the optical coupler 12 to the photodiode 22 for capturing the reference band and the signal band wavelengths. Light 47 is emitted from the GBP through the fiber 28 and transmitted toward the fiber lens 34. The light 47 then travels through the fiber lens 34 which focuses the light 47 and directs it toward the mirrored surface 39 which reflects the light 47 toward the first dichroic coating 42 which transmits the light 47 toward the second dichroic coating 44.

The above defined fiber optic CGM device 10 can be housed within a cover 25, as described previously, or modified to utilize the illustrative OBS covers described below. Additionally, alternative optical CGM devices known in the art can also be modified to include the illustrative OBS covers described below.

An OBS cover encloses and protects the CGM device 10 from environmental conditions that may adversely affect and/or damage the components of the CGM device 10.

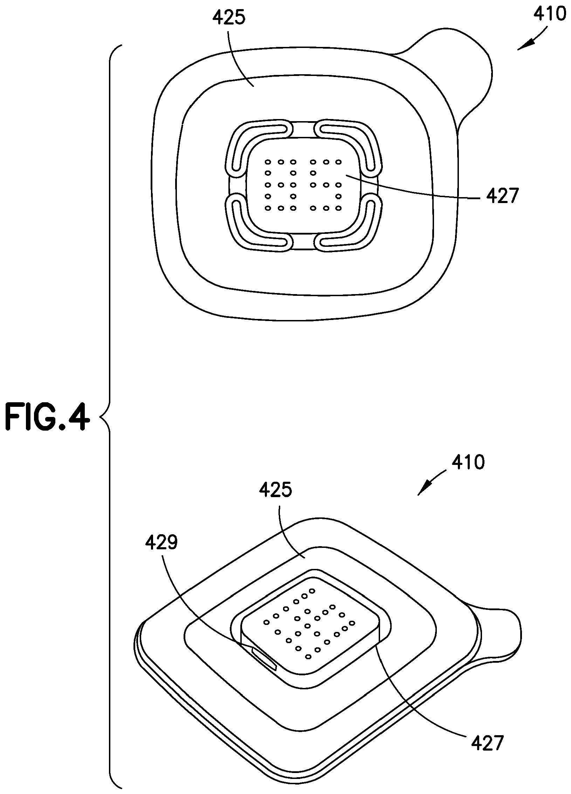

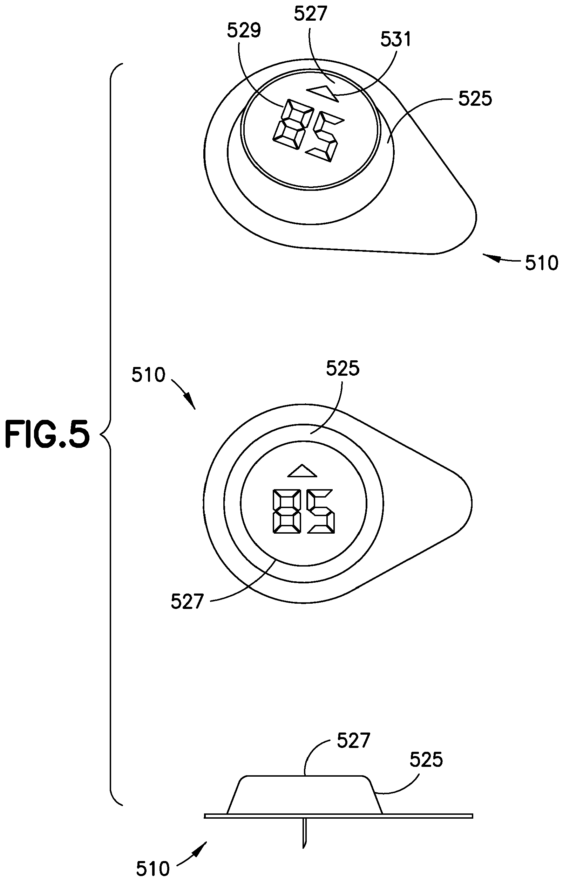

Fig. 4 illustrates a top and perspective view of an illustrative embodiment of a CGM sensor 410 in accordance with the present invention. The CGM sensor 410 has profile shape that is lower than that of the CGM sensor 10, but can operate in substantially the same way. The CGM sensor 410 includes an output display 427 integral with and does not extend substantially higher than the cover 425. The output display 427 is adapted to provide a user with visual confirmation of a user's current glucose levels, glucose trends, CGM device malfunction notifications, glucose measurement intervals, and any other desired notifications. The device 410 may also include a gyroscope 429 such that it will recognize its orientation and output correctly oriented information on display 427 so that a user can correctly view the output information. Providing a gyroscope 429 to the device 410 can be a significant benefit when the CGM device is placed in alternative locations on the user's body. The output display 427 can display, for example, digital representation of data or more analog representation of data where small LEDs or lights illuminate a pattern representing the output data, similar to the illustrative embodiment shown in Fig. 5. Alternative displays can also include a liquid-crystal display (LCD), a thermometer graph or a speedometer graph.

In an illustrative embodiment in accordance with the present invention, as shown in Fig. 4, integrating the output display 427 into the cover 425 can be accomplished without significantly increasing the profile of the CGM device 410. Keeping a reduced profile is important because a CGM with a lower profile is less likely to irritate or interfere with a user's everyday activities.

Fig. 5 illustrates an illustrative embodiment of an on-body CGM device 510 in accordance with the present invention, which includes a cover 525 with an integrated output display 527. The output display 527 includes a digital readout and illustrates an example of a numerical reading 529 and a current trend indication (upward arrow) 531. As shown in Fig. 5 the output display does not significantly add to the profile of the CGM device, thus minimizing interference with a user during use.

Other illustrative embodiments of output displays can include pop-up type displays, mirrored surface displays, tethered displays having a coiled connection with the CGM device and displays oriented on a side surface of the cover as opposed to a top surface as previously disclosed. Other illustrative embodiments of displays can also include modular displays that are removable from the CGM device cover. Modular displays, for example, can be magnetically secured to the cover or mechanically secured using a snap fit engagement, rail locking mechanism, disconnectable tether or adhesive. For example, the display can include an e-ink paper display with adhesive backing.

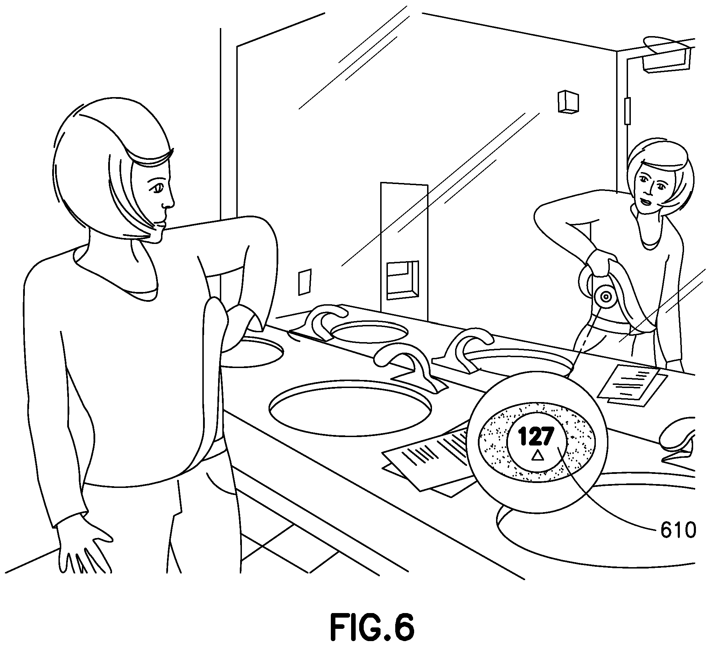

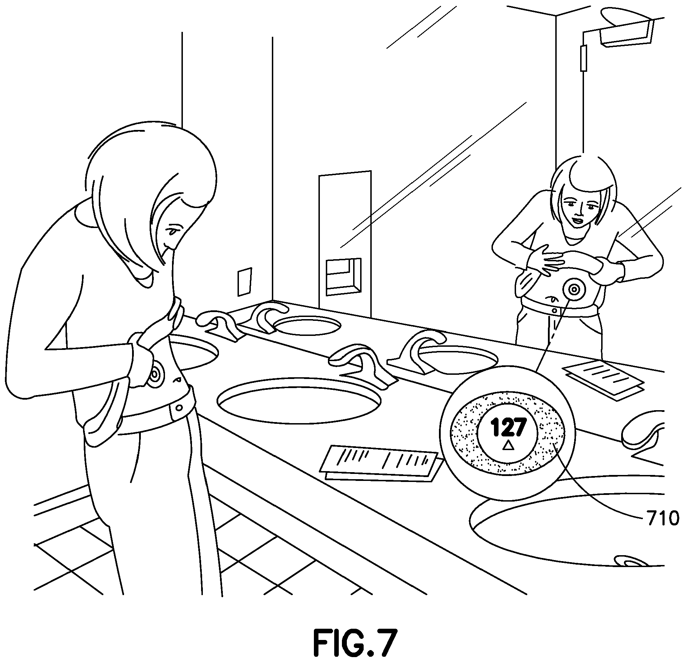

Figs. 6 and 7 illustrate how on-body displays for CGM devices 610 and 710 can be inspected by a user in an everyday-type situation, as well as, illustrating the convenience of having a visual on-body display for the CGM devices 610 and 710.

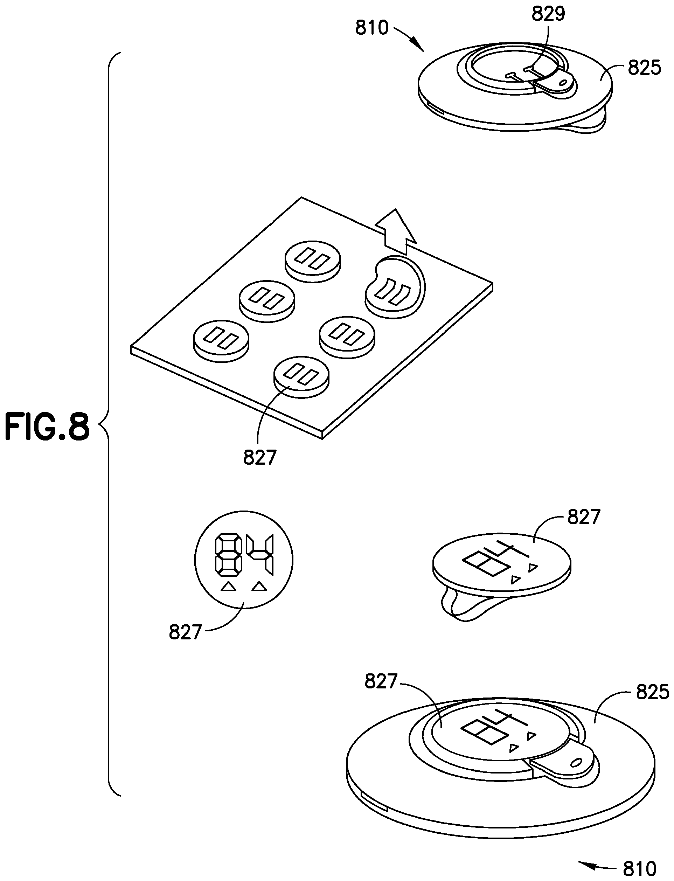

Fig. 8 illustrates a further illustrative embodiment of an on-body CGM device 810, which includes a cover 75 having a recess 829 for receiving an output display 827. The recess 829 in the cover 825 aids in maintaining a low profile when the display is secured to the cover 825. The output display 827 is preferably formed using a thin e-paper material. The microcontroller can include, but is not limited to, microcontroller 21 of the illustrative embodiment of the CGM sensor 10.

In an alternative illustrative embodiment in accordance with the present invention, microcontroller 21 can include a transceiver compatible with a transceiver integral with the e-paper display 77. Thus the microcontroller 21 can transmit data to be displayed by the e-paper display 77. Other alternative transmission systems can also be used to transmit data from the microcontroller to the e-paper display 77. Medical devices currently use radio frequency (RF) wireless communications such as Bluetooth®, Zigbee®, 802.11, or other conventional solutions. Some medical devices even communicate via a line-of-sight using infrared (IR) technology. Wireless communication systems, since they do not require a line of sight, are preferred over IR technology.

Conventional wireless technology is a driving contributor in the cost of medical devices that use their respective technologies. Advantageously, in an alternative illustrative embodiment in accordance with the present invention, the illustrative embodiment shown in Fig. 8 may also be configured to use a capacitively coupled personal area network (PAN) to transceive data between microcontroller 21 and the e-paper display 827 through the user's skin, without the use of antennas. A personal area network, can be created with simple, low-cost microcontrollers and analog components, requires less power to operate than RF systems and are at least as secure as RF systems. The use of a personal area network can enable extended use duration due to the reduced component cost and lower power requirements.

In an illustrative embodiment in accordance with the present invention, a PAN transceiver can be integrated with the microcontroller 21 to establish a personal area network to communicate with the output display 827 via a "near field" electric field that transmits data using the human body as a transport medium. The microcontroller 21 and the output display each need PAN transceivers, respectively, in order to communicate to each other through the body.

In an illustrative embodiment in accordance with the present invention, a PAN communication system ensures that only people in direct contact with a user are capable of detecting the signals propagating across the user's body. Alternatively, in conventional wireless technologies, a transmitted signal can be detected by anyone with a receiver in the respective range of the wireless technology. The necessary transceiver components for realizing the functionality of the illustrative personal area network discussed above, are widely available and relatively low in cost.

In an illustrative embodiment in accordance with the present invention, utilizing the PAN communication system can enable a user to secure the e-paper display 827 on an alternative area on a user's body, separate from the CGM device. E-paper displays 827 can also be relatively inexpensive and thus, disposable after short term use, enabling a user to replace the e-paper displays after exercising, for example.

Although only a few illustrative embodiments of the present invention have been described in detail above, those skilled in the art will readily appreciate that many modifications are possible in the illustrative embodiments, and various combinations of the illustrative embodiments are possible, without materially departing from the novel teachings and advantages of this invention. Accordingly, all such modifications are intended to be included within the scope of this invention.

## Claims

1. An on-body device (10) for sensing an analyte in a living body, comprising:
a cover (25) at least partially containing a continuous glucose monitoring sensor having a microcontroller;
a first bottom surface (18) adapted to be adhered to skin; and
a second surface having a display (27) adapted to display data transmitted by the microcontroller
**characterized by** a gyroscope (429) adapted to recognize an orientation of the gyroscope, wherein the microcontroller is configured to control the display to display information in accordance with the recognized orientation of the gyroscope.

2. The on-body device of claim 1, wherein the data comprises data relating to at least one selected from the set consisting of:
a user's current glucose levels;
glucose trends;
CGM device malfunction notifications;
when the output display is illuminated or shut down; and
glucose measurement intervals.

3. The on-body device of claim 1, wherein the second surface comprises at least one selected from the set consisting of a top surface of the cover and a side surface of the cover.

4. The on-body device of claim 1, wherein the display displays at least one selected from the set consisting of:
a digital representation of data; and
an analog representation of data.

5. The on-body device of claim 1, wherein the display (27) comprises at least one display element selected from the set consisting of:
a light-emitting diode (24); and
a liquid-crystal display;
wherein the at least one display element is adapted to illuminate a pattern representing output data.

6. The on-body device of claim 1, wherein the display comprises at least one display element selected from the set consisting of:
a thermometer graph; and
a speedometer graph.

7. The on-body device of claim 1, wherein the display does not extend substantially higher than the cover.

8. The on-body device of claim 1, wherein the display is adapted to display a trend indication.

9. The on-body device of claim 8, wherein the trend indication comprises an arrow.

10. The on-body device of claim 1, wherein the cover has a recess adapted to receive the display.

11. The on-body device of claim 1, wherein the display comprises an e-paper material.

12. The on-body device of claim 1, wherein the microcontroller comprises a transceiver compatible with a transceiver integral with the display.

13. The on-body device of claim 1, wherein the microcontroller is adapted to communicate with the display using at least one selected from the set consisting of:
radio frequency radio communication;
line-of-sight communication;
a personal area network; and
near field communication.

14. The on-body device of claim 1, wherein the display is replaceable or removable.

## Patentansprüche

1. Am Körper tragbare Vorrichtung (10) zum Sensieren eines Analyten in einem lebenden Körper mit:
einer Abdeckung (25), die zumindest teilweise einen Sensor zur kontinuierlichen Blutzuckerüberwachung mit einem Mikrocontroller beinhaltet;
einer ersten Unterfläche (18) zum Anhaften auf Haut; und
einer zweite Fläche mit einem Display (27) zum Anzeigen von durch den Microcontroller gesendeten Daten,
**gekennzeichnet durch**
ein Gyroskop (429) zum Erkennen einer Orientierung des Gyroskops, wobei der Microcontroller zum Steuern des Displays ausgebildet ist, um Informationen entsprechend der erkannten Orientierung des Gyroskops anzuzeigen.

2. Am Körper tragbare Vorrichtung nach Anspruch 1, wobei zu den Daten solche Daten zählen, die sich auf mindestens eines beziehen, das ausgewählt ist aus der Gruppe bestehend aus Folgendem:
aktuelle Blutzuckerwerte eines Benutzer;
Blutzuckertendenzen;
Störmeldungen der Vorrichtung zur kontinuierlichen Blutzuckerüberwachung;
Zeitpunkt zu dem das Ausgangsdisplays beleuchtet oder ausgeschaltet ist; und
Blutzuckermessintervalle.

3. Am Körper tragbare Vorrichtung nach Anspruch 1, wobei die zweite Fläche mindestes eines aufweist, das ausgewählt ist aus der Gruppe bestehend aus einer oberen Fläche der Abdeckung und einer Seitenfläche der Abdeckung.

4. Am Körper tragbare Vorrichtung nach Anspruch 1, wobei das Display mindestens eines anzeigt, das ausgewählt ist aus der Gruppe bestehend aus:
einer digitalen Datendarstellung; und
einer analogen Datendarstellung.

5. Am Körper tragbare Vorrichtung nach Anspruch 1, wobei das Display (27) mindestens ein Displayelement aufweist, das ausgewählt ist aus der Gruppe bestehend aus:
einer Leuchtdiode (24); und
einem Flüssigkristalldisplay;
wobei das mindestens eine Displayelement ein Muster beleuchtet, das Ausgangsdaten darstellt.

6. Am Körper tragbare Vorrichtung nach Anspruch 1, wobei das Display mindestens ein Displayelement aufweist, das ausgewählt ist aus der Gruppe bestehend aus:
einem Thermometergraphen; und
einem Geschwindigkeitsmessungsgraphen.

7. Am Körper tragbare Vorrichtung nach Anspruch 1, wobei sich das Display im Wesentlichen nicht höher als die Abdeckung erstreckt.

8. Am Körper tragbare Vorrichtung nach Anspruch 1, wobei das Display eine Tendenzangabe anzeigt.

9. Am Körper tragbare Vorrichtung nach Anspruch 8, wobei die Tendenzangabe einen Pfeil aufweist.

10. Am Körper tragbare Vorrichtung nach Anspruch 1, wobei die Abdeckung eine Aussparung zum Aufnehmen des Displays aufweist.

11. Am Körper tragbare Vorrichtung nach Anspruch 1, wobei das Display ein E-Paper-Material aufweist.

12. Am Körper tragbare Vorrichtung nach Anspruch 1, wobei der Microcontroller einen Sende-Empfänger aufweist, der mit einem Sende-Empfänger kompatibel ist, der einstückig mit dem Display ausgebildet ist.

13. Am Körper tragbare Vorrichtung nach Anspruch 1, wobei der Microcontroller zum Kommunizieren mit dem Display ausgebildet ist mittels mindestens einem, das ausgewählt ist aus der Gruppe bestehend aus Folgendem:
Radiofrequenz-Funkkommunikation;
Sichtkommunikation
einem Personal Area Network; und
Nahfeldkommunikation.

14. Am Körper tragbare Vorrichtung nach Anspruch 1, wobei das Display austauschbar oder entfernbar ist.

## Revendications

1. Dispositif porté sur le corps (10) pour détecter un analyte dans un corps vivant, comprenant :
un couvercle (25) contenant au moins partiellement un capteur de surveillance continue de glucose ayant un microcontrôleur ;
une première surface inférieure (18) adaptée pour adhérer à la peau ; et
une deuxième surface ayant un afficheur (27) adapté pour afficher des données transmises par le microcontrôleur
**caractérisé par**
un gyroscope (429) adapté pour reconnaître une orientation du gyroscope, où le microcontrôleur est configuré pour commander l'afficheur pour afficher des informations conformément à l'orientation reconnue du gyroscope.

2. Dispositif porté sur le corps de la revendication 1, dans lequel les données comprennent des données relatives à au moins un élément choisi dans l'ensemble constitué par :
les taux de glucose actuels d'un utilisateur ;
les tendances glycémiques ;
les notifications de dysfonctionnement du dispositif CGM ;
le moment auquel l'afficheur de sortie est allumé ou éteint ; et
les intervalles de mesure du glucose.

3. Dispositif porté sur le corps de la revendication 1, dans lequel la deuxième surface comprend au moins une surface choisie dans l'ensemble constitué par une surface supérieure du couvercle et une surface latérale du couvercle.

4. Dispositif porté sur le corps de la revendication 1, dans lequel l'afficheur affiche au moins une représentation choisie dans l'ensemble constitué par :
une représentation numérique des données ; et
une représentation analogique des données.

5. Dispositif porté sur le corps de la revendication 1, dans lequel l'afficheur (27) comprend au moins un élément d'affichage choisi dans l'ensemble constitué par :
une diode électroluminescente (24) ; et
un afficheur à cristaux liquides ;
dans lequel l'au moins un élément d'affichage est adapté pour éclairer un motif représentant des données de sortie.

6. Dispositif porté sur le corps de la revendication 1, dans lequel l'afficheur comprend au moins un élément d'affichage choisi dans l'ensemble constitué par :
un graphique de thermomètre ; et
un graphique d'indicateur de vitesse.

7. Dispositif porté sur le corps de la revendication 1, dans lequel l'afficheur ne s'étend pas à une hauteur essentiellement plus élevée que le couvercle.

8. Dispositif porté sur le corps de la revendication 1, dans lequel l'afficheur est adapté pour afficher une indication de tendance.

9. Dispositif porté sur le corps de la revendication 8, dans lequel l'indication de tendance comprend une flèche.

10. Dispositif porté sur le corps de la revendication 1, dans lequel le couvercle a un évidement adapté pour recevoir l'afficheur.

11. Dispositif porté sur le corps de la revendication 1, dans lequel l'afficheur comprend une matière de papier électronique.

12. Dispositif porté sur le corps de la revendication 1, dans lequel le microcontrôleur comprend un émetteur-récepteur compatible avec un émetteur-récepteur intégré à l'afficheur.

13. Dispositif porté sur le corps de la revendication 1, dans lequel le microcontrôleur est adapté pour communiquer avec l'afficheur en utilisant au moins un élément choisi dans l'ensemble constitué par :
une radiocommunication radiofréquence ;
une communication en visibilité directe ;
un réseau personnel ; et
une communication en champ proche.

14. Dispositif porté sur le corps de la revendication 1, dans lequel l'afficheur est remplaçable ou amovible.
